# EUROPEAN PATENT APPLICATION

(11) **EP 2 060 230 A1**
(43) Date of publication of application: **20.05.2009**
(21) Application number: 08168891.3
(22) Date of filing: 12.11.2008
(51) Int. Cl.: A61B 5/11

(54) **Bed apparatus and method of determining body movement**

(30) Priority: 13.11.2007 JP 2007294008
(71) Applicant: M.I. Laboratories Corporation, Shinagawa-ku, Tokyo 141-0001 (JP)
(72) Inventor: Takashima, Mitsuru, Shinagawa-ku Tokyo 141-0001 (JP)
(74) Representative: Schwabe - Sandmair - Marx

(57) **Abstract**

A bed apparatus includes a connecting frame of a bed, a detecting unit, a determining unit, and an output unit. The detecting unit is provided on a part of the connecting frame, and detects an amount of deformation of the connecting frame continuously. The determining unit determines a body movement of a person on the bed based on a result of detection by the detecting unit. The output unit outputs a result of determination made by the determining unit.

## Description

### BACKGROUND OF THE INVENTION

### 1. Field of the Invention

The present invention relates to a bed apparatus and a method of determining body movement of a person on the bed apparatus, both of which are used at hospitals and nursing-care facilities.

### 2. Description of the Related Art

Nowadays, hospitals and nursing-care facilities are required to monitor patients in bed. Therefore, the movement of a person on a bed is detected and centralized management is performed to reduce the work of nurses and to ensure the safety of patients. Video monitoring is the simplest and the most accurate method to detect movement. However, with such a method, monitoring is difficult when the room is dark. Furthermore, it is inappropriate from the viewpoint of privacy. For this reason, a method of detecting the movement of a person on a bed without restricting movement and without conscious awareness of such monitoring by the person is demanded.

For example, an apparatus that has a body movement sensor including a weight sensor and piezoelectric film disposed on a bed has been disclosed (for example, Japanese Patent No. 2806214). The apparatus detects the presence or absence of a person based on a change in detected weight, detects a rolling movement of the person by the piezoelectric film, and detects breathing and heartbeat to report an abnormal state of health.

However, for the technique disclosed in Japanese Patent No. 2806214, it is an absolute requirement that a person be on the sensor, and if relative positions change, the various types of detection cannot be performed. Furthermore, since the sensor is used close to the body of a person, waterproofing and electrical insulating are essential to protect the sensor from perspiration and urine. In addition, since a person lies on the sensor for a long time, body pressure is not distributed and a pressure sore can result.

It is an object of the present invention to solve problems related to the conventional technique described above and to provide a bed apparatus that can easily detect body movement of a person on a bed without causing an unpleasant feeling, restricting movement, or conscious awareness of such monitoring by the person.

### SUMMARY OF THE INVENTION

It is an object of the present invention to at least solve the above problems in the conventional technologies.

A bed apparatus according to one aspect of the present invention includes a frame of a bed; a deformation-amount detecting unit that is disposed on a part of the frame and continuously detects an amount of deformation of the frame; a determining unit that determines a body movement of a person on the bed based on a result of detection by the deformation-amount detecting unit; and an output unit that outputs a result of determination made by the determining unit.

A body movement determining method according to another aspect of the present invention is for a bed apparatus that includes a frame of a bed and a deformation-amount detecting unit disposed on a part of the frame. The body movement determining method includes detecting continuously an amount of deformation of the frame; determining a body movement of a person on the bed based on a result of detection at the detecting; and outputting a result of determination made by the determining unit.

The other objects, features, and advantages of the present invention are specifically set forth in or will become apparent from the following detailed description of the invention when read in conjunction with the accompanying drawings.

### BRIEF DESCRIPTION OF THE DRAWINGS

Fig. 1 is a system configuration diagram of a bed apparatus according to an embodiment of the present invention;
Fig. 2 is an enlarged view of the bed apparatus near a unimorph device;
Fig. 3A illustrates a detailed configuration of the unimorph device;
Fig. 3B is a cross-section of the unimorph device when deformed;
Fig. 3C is another cross-section of the unimorph device when deformed;
Fig. 4 is a block diagram of a determining mechanism;
Fig. 5 is a block diagram of the bed apparatus,
Fig. 6 is a graph of a voltage signal output from the unimorph device;
Fig. 7 is a graph of the voltage signal output from the unimorph device;
Fig. 8A is a flowchart of processing performed by the bed apparatus when a person gets on a bed;
Fig. 8B is a flowchart of processing performed by the bed apparatus when a movement of a person is to be determined;
Fig. 8C is a flowchart of processing performed by the bed apparatus when a person gets off the bed;
Fig. 9 is a block diagram of another determining mechanism;
Fig. 10 is a graph of a voltage signal output from the unimorph device;
Fig. 11 is a flowchart of processing performed by the bed apparatus according to a second embodiment; and
Fig. 12 is a flowchart of processing performed by the bed apparatus according to a third embodiment.

### DETAILED DESCRIPTION OF THE PREFERRED EMBODIMENTS

Exemplary embodiments of the present invention are explained in detail below with reference to the accompanying drawings.

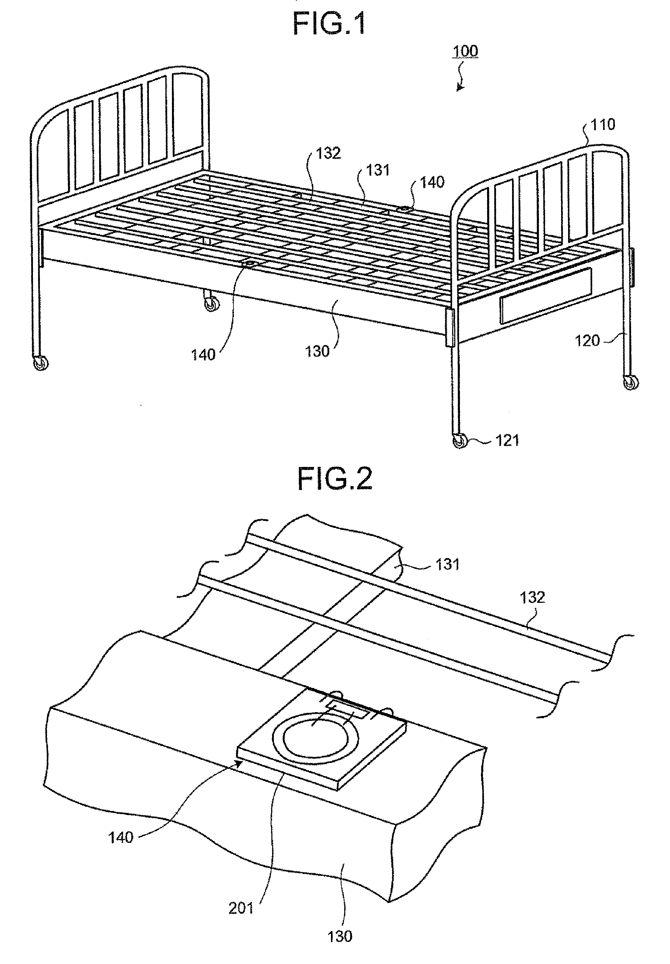

First, a configuration of a bed apparatus 100 according to an embodiment of the present invention is explained. Fig. 1 is a system configuration diagram of the bed apparatus 100 according to the embodiment. As shown in Fig. 1, the bed apparatus 100 includes a bed 110 and a unimorph device 140.

Provided a frame thereof is rigid, the bed 110 can be made of, for example wood. However, generally, one made of metal is used because hysteresis with respect to stress is low and change over time is minimal. The bed 110 is, for example, a bed that is used at hospitals or the like.

The bed 110 includes legs 120 and connecting frames 130 (corresponding to connecting members). A caster 121 is provided on each of the legs 120, to transport a patient. The connecting frames 130 connect four pieces of the legs 120. A joint 131 connects, in a direction of width, one pair of the connecting frames 130 that are arranged in a direction of length. Connecting lines 132 connect, in the direction of length, another pair of the connecting frames 130 that are arranged in the direction of width, and form a base plate.

The unimorph device 140 corresponds to a deformation-amount detecting unit, and is disposed at the center of each of the connecting frames 130 that stretch along the direction of length. The unimorph device 140 detects distortion of the connecting frames 130 caused when a person gets on and off the bed 110 or caused by other body movements.

Next, details of the unimorph device 140 are explained with reference to Fig. 2. Fig. 2 is an enlarged view of the bed apparatus 100 shown in Fig. 1, near the unimorph device 140.

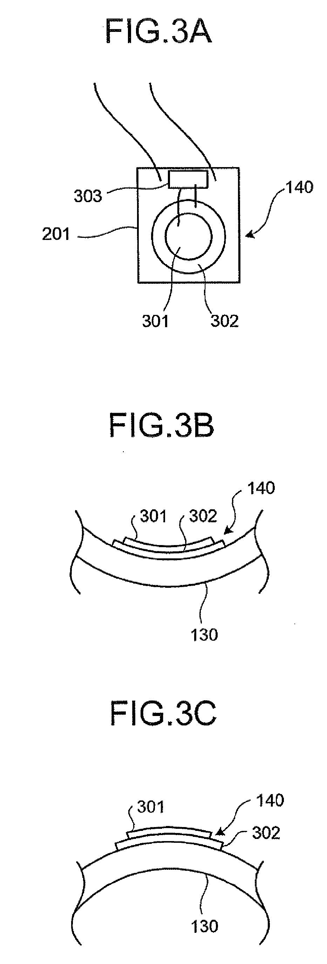

As shown in Fig. 2, the unimorph device 140 is fixed to a substrate 201 of a rectangular shape. This substrate 201 is disposed on the connecting frame 130. Thus, the unimorph device 140 is mounted on the connecting frame 130. The unimorph device 140 has a configuration in which a piezoelectric device having a thin plate shape and a metallic plate are laminated to each other, and outputs a voltage that corresponds to the amount of deformation of the metallic plate when the metallic plate is deformed along with deformation of the substrate 201. Further details are explained hereinafter with reference to Fig. 3. Since the unimorph device 140 is a conventional technology, detailed explanation thereof is omitted herein.

As the deformation-amount detecting unit, a bimorph device can be used in place of the unimorph device 140. A bimorph device has a configuration such that a piezoelectric device having a thin plate shape is laminated to both sides of a metallic plate, and outputs a voltage that corresponds to the amount of deformation when the connecting frame 130 is deformed. Since the bimorph device is also a conventional technology, detailed explanation thereof is omitted herein.

Moreover, as the deformation-amount detecting unit, a pressure sensitive device that outputs a voltage corresponding to the stress imparted thereto can be used in place of the unimorph device 140, such as a piezoelectric device among a variety of types (excluding the unimorph device 140), a semiconductor, or a resistor strain sensor. Since such pressure sensitive devices are also conventional technologies, detailed explanation thereof is omitted herein.

The mounting position of the unimorph device 140 is not limited, provided that the unimorph device 140 can detect distortion of the connecting frame 130 of the bed 110. However, it is preferable that the unimorph device 140 be mounted at substantially the center of the connecting frame 130 with respect to the direction of length, a position at which the distortion can be detected most. In recent years, beds that can move the head and legs of a person by tilting are commonly used, and the bed 110 can take such a form. Since a structure of the connecting frame 130 or the like is complicated in such a bed, an appropriate number of the unimorph devices 140 should be provided at appropriate positions so as to detect the distortion of the connecting frame 130.

Next, a detailed configuration of the unimorph device 140 is explained with reference to Fig. 3A. Fig. 3A illustrates a detailed configuration of the unimorph device 140.

As shown in Fig. 3A, the unimorph device 140 is mounted in such a state that the entire surface of a disc-shaped metallic plate 302, on which a disc-shaped piezoelectric device 301 is laminated, is fixed to the substrate 201. Thus, distortion of the substrate 201 can be transmitted well to the metallic plate 302 of the unimorph device 140. When the piezoelectric device 301 and the metallic plate 302 are formed in a disc shape, distortion in any direction can be detected. Therefore, the mounting direction of the unimorph device 140 is not limited, and the unimorph device 140 can be mounted easily.

An amplifier 303 that amplifies a voltage output from the unimorph device 140 is provided on the substrate 201. The amplifier 303 is implemented by, for example, an impedance conversion device. The impedance conversion device performs impedance conversion of the voltage output from the unimorph device 140. Impedance is a ratio of voltage and current in an alternating circuit. As the impedance conversion device, for example, a high-input impedance conversion device such as a field effect transistor (FET) can be used.

In the present embodiment, the unimorph device 140 whose capacitance is 10 nanofarads to 20 nanofarads is used, and for example, the unimorph device 140 whose capacitance is 16 nanofarads is used. The amplifier 303 is configured to have input impedance of 100 megaohms (MΩ) to perform the impedance conversion and to amplify a voltage after the impedance conversion. Therefore, it is possible to detect a signal of a low frequency band accurately.

Next, an example of a case when the unimorph device 140 is deformed is explained with reference to Figs. 3B and 3C. Figs. 3B and 3C are cross-sections of the unimorph device 140 when the unimorph device 140 is deformed.

Fig. 3B illustrates the unimorph device 140 in a concave state. For example, this indicates distortion of the connecting frame 130 when a person gets on the bed 110. Such a concave state is referred to as "bend". This bend is caused also when a person changes a position on the bed 110. While the bend is emphasized in Fig. 3B, only a slight bend is caused actually.

Fig. 3C illustrates the unimorph device 140 in a convex state. For example, this indicates distortion of the connecting frame 130 caused by rebound, i.e., resiliency thereof, after a person gets off the bed 110. Such a convex state is referred to as "rebound-warp". While the rebound-warp is emphasized in Fig. 3C, only slight rebound-warp is caused actually.

The unimorph device 140 is deformed as a result of a slight deformation of the connecting frame 130, and outputs a voltage corresponding to the amount of the deformation. Furthermore, on the substrate 201, in addition to the unimorph device 140, a determining mechanism is provided that determines a getting on/off the bed 110 of a person based on the voltage output from the unimorph device 140.

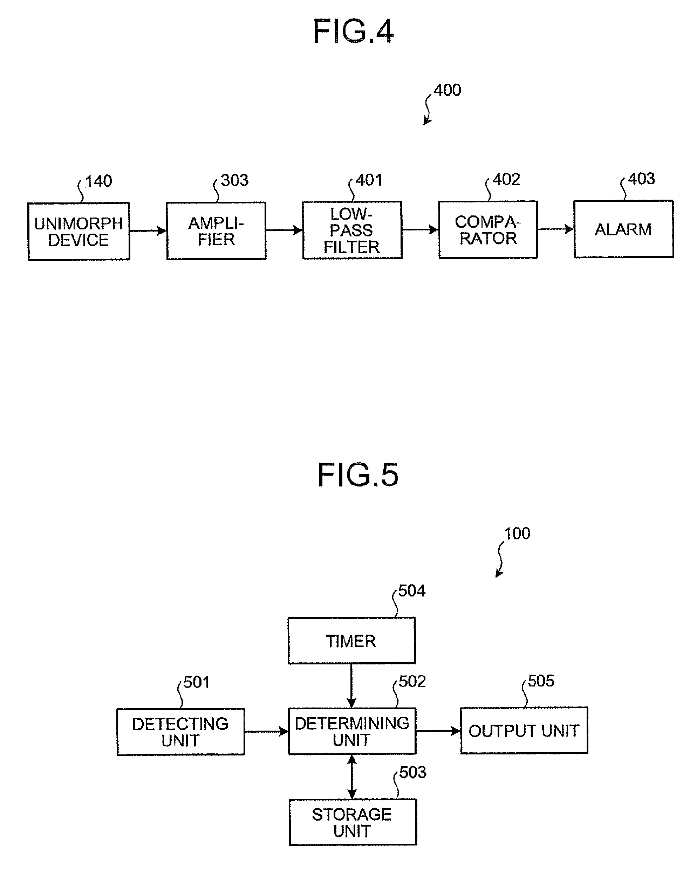

Next, a functional configuration of a determining mechanism 400 is explained with reference to Fig. 4. Fig. 4 is a block diagram of the determining mechanism 400. As shown in Fig. 4, the determining mechanism 400 includes the unimorph device 140, the amplifier 303, a low-pass filter 401, a comparator 402, and an alarm 403.

As described above, the unimorph device 140 is deformed as a result of a slight deformation of the connecting frame 130, and outputs a voltage corresponding to the amount of the deformation. When a person gets on the bed 110, for example, the unimorph device 140 provided on top of the connecting frame 130 is bent, and positive voltage is generated from the piezoelectric device 301. The positive voltage is inverted by the amplifier 303 (impedance conversion device) to a negative voltage. Therefore, when the unimorph device 140 is bent, negative voltage is extracted.

On the other hand, when a person gets off the bed 110 and the unimorph device 140 is warped, negative voltage is generated from the piezoelectric device 301. The negative voltage is inverted by the amplifier 303 (impedance conversion device) to a positive voltage. Therefore, when the unimorph device 140 is warped, positive voltage is extracted.

If the unimorph device 140 is provided at the bottom of the connecting frame 130, the reverse voltage is extracted. Specifically, when a person gets on the bed 110, positive voltage is extracted, and when a person gets off the bed 110, negative voltage is extracted.

From the voltage amplified by the amplifier 303, the low-pass filter 401 filters for components of 0.1 hertz (Hz) to 1 Hz, which pass to the comparator 402. Thus, components originating from the getting on/off the bed 110 of a person and components originating from a movement of a person on the bed 110 can be extracted from components of the voltage amplified by the amplifier 303.

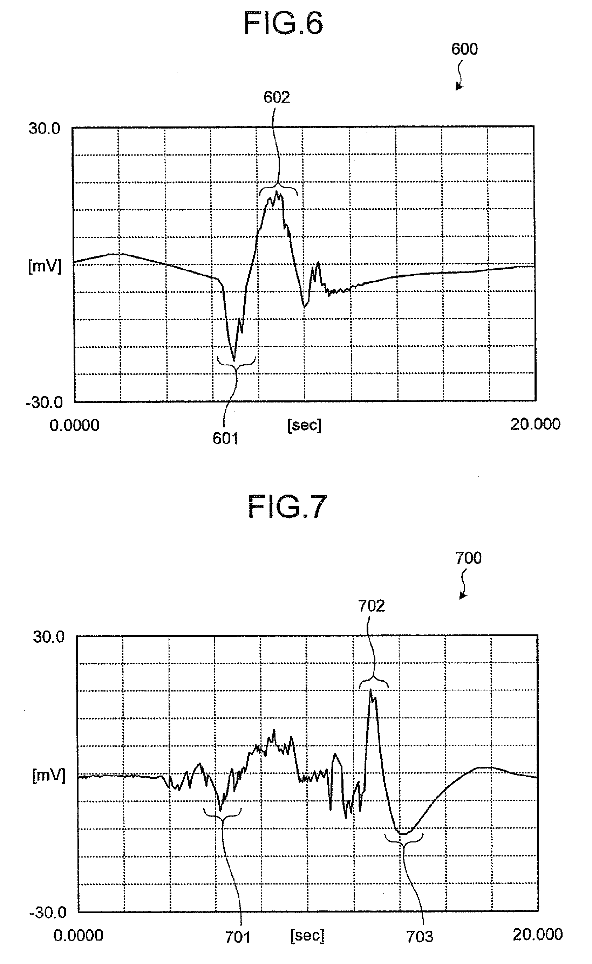

The comparator 402 has a storage area (not shown) to store a threshold used for comparison, and compares the threshold stored in the storage area and a voltage signal that is obtained by filtering with the low-pass filter 401. The comparator 402 sends a signal corresponding to a result of the comparison to the alarm 403 downstream. The threshold is set in advance based on waveform patterns (refer to Figs. 6 and 7) that are expressed by voltage over time upon a person getting on/off the bed 110 and a movement of a person on the bed 110. The threshold is, specifically, expressed by an amplitude and a duration of the voltage signal.

While the threshold is explained in detail hereinafter with reference to Figs. 6 and 7, as for getting on and off the bed 110, for example, the threshold is set as "maximum amplitude: ±12 millivolts (mV)" and "duration: 2 seconds (sec)". As for a movement of a person on the bed 110, the threshold is set as "maximum amplitude: -6 mV" and "duration: 2 sec".

For getting on and off the bed 110, for example, the comparator 402 compares the threshold (for example, ±12 mV) and a voltage signal obtained by filtering, and when a peak whose absolute value of the maximum amplitude is 12 mV or higher is present in the voltage signal, the comparator 402 sends, to the alarm 403 downstream, a signal that indicates such accordingly. Furthermore, the comparator 402 compares duration of the peak whose absolute value of the maximum amplitude is 12 mV or higher with the duration of the threshold peak (2 sec), and when it is determined that the peak is within 2 sec as a result of the comparison, the comparator 402 sends, to the alarm 403 downstream, a signal that indicates such accordingly.

Moreover, for a movement of a person on the bed 110, the comparator 402 compares the threshold (for example, -6 mV) and a voltage signal obtained by filtering, and when a peak whose maximum amplitude is -6 mV or lower is present, the comparator 402 sends, to the alarm 403 downstream, a signal that indicates such accordingly. Furthermore, the comparator 402 compares the duration of the peak whose maximum amplitude is -6 mV or lower with the duration of the threshold peak (2 sec), and when it is determined that the peak is within 2 sec as a result of the comparison, the comparator 402 sends, to the alarm 403 downstream, a signal that indicates such accordingly.

For simplicity, in this example, the result of detection is obtained based on an output value of only one of the unimorph devices 140 that outputs the voltage described above, that is, it is determined that a getting on/off the bed 110 of a person or a movement of a person on the bed 110 is detected when at least one of the unimorph devices 140 outputs the voltage described above. However, if the unimorph device 140 is provided on each of the connecting frames 130, a result of detection can be obtained by performing a difference operation on both output values.

In this case, a threshold to be used for comparison when the difference operation is performed can be stored, and a value obtained by the difference operation can be compared with the threshold. By thus performing the difference operation, it becomes possible to determine a body movement of a person in more detail, particularly, in a bed having a complicated frame structure.

The alarm 403 outputs an alarm signal based on the signal sent from the comparator 402. For example, when the alarm 403 receives, from the comparator 402, a signal indicating that a voltage signal of -12 mV has been output, the alarm 403 sends an alarm signal to an indicator lamp (not shown), thereby lighting the indicator lamp to indicate that a person has got on the bed 110. Such an indicator lamp is provided, for example, in a management room from which patients are managed in a hospital.

Moreover, for example, when the alarm 403 receives, from the comparator 402, a signal indicating that a voltage signal of +12 mV has been output, the alarm 403 sends an alarm signal to an indicator lamp (not shown), thereby lighting the indicator lamp to indicate that a person has got off the bed 110.

For example, when the alarm 403 receives, from the comparator 402, a signal indicating that a voltage signal of -6 mV has been output, the alarm 403 sends an alarm signal to an indicator lamp (not shown), thereby lighting the indicator lamp to indicate that a person on the bed 110 has move to an edge of the bed 110.

The amplifier 303, the low-pass filter 401, the comparator 402, and the alarm 403 in the determining mechanism 400 can be implemented by a microcomputer that is configured with a read only memory (ROM) that stores programs to realize each function, a central processing unit (CPU) that executes various kinds of arithmetic processings based on the programs stored in the ROM, a random access memory (RAM) that functions as a work area of data for the CPU to perform an arithmetic processing, and the like.

Each function of the amplifier 303, the low-pass filter 401, the comparator 402, and the alarm 403 is not limited to implementation by a single microcomputer, and each function can be implemented, for example, by a combination of microcomputers (circuits) that separately realize each function.

Next, a functional configuration of the bed apparatus 100 according to the present invention is explained with reference to Fig. 5. Fig. 5 is a block diagram of the bed apparatus 100. As shown in Fig. 5, the bed apparatus 100 includes a detecting unit 501, a determining unit 502, a storage unit 503, a timer 504, and an output unit 505.

The detecting unit 501 detects the amount of deformation of the connecting frame 130 of the bed 110. Specifically, as the amount of deformation of the connecting frame 130, the detecting unit 501 detects the voltage signal that is obtained by amplifying a voltage signal output from the unimorph device 140 by the amplifier 303 and by filtering the voltage signal by the low-pass filter 401. The function of the detecting unit 501 can be implemented by the unimorph device 140, the amplifier 303, and the low-pass filter 401 shown in Fig. 4.

In other words, the function of the detecting unit 501 is implemented when the CPU in the microcomputer configuring the determining mechanism 400 shown in Fig. 4 executes a program, from among the programs stored in a memory such as the ROM and the RAM in the microcomputer, relating to the detection of the amount of deformation.

The determining unit 502 determines whether the deformation of the connecting frame 130 exceeds a threshold based on the result of detection by the detecting unit 501 and the threshold stored in the storage unit 503. Specifically, the determining unit 502 determines whether fluctuation of the voltage signal obtained as an amount of deformation of the connecting frame 130 is equal to or exceeds the threshold. The storage unit 503 is implemented, specifically, by a memory such as the ROM and the RAM in the microcomputer, for example. The determining unit 502 determines that the amount of deformation of the connecting frame 130 is equal to or exceeds the threshold when a pulsed signal is output from the comparator 402.

Moreover, the determining unit 502 determines whether the voltage signal indicating the amount of deformation of the connecting frame 130 fluctuates as much as the threshold or more within a predetermined time based on time counted by the timer 504. Specifically, the function of the timer 504 is implemented by, for example, a timer function of the CPU in the microcomputer configuring the determining mechanism 400 shown in Fig. 4.

For example, when a pulsed signal is output from the comparator 402, the determining unit 502 determines whether duration of a peak being a trigger to output the pulsed signal is within the predetermined time (duration for comparison). If the duration of the peak is within the predetermined time, the determining unit 502 determines whether the fluctuation of voltage in the peak exceeds the threshold.

Specifically, with regard to a getting on/off the bed 110 of a person, for example, the determining unit 502 determines that the voltage signal indicating the amount of deformation of the connecting frame 130 exceeds the threshold when, as a result of comparison performed by the comparator 402, it is found that a peak is present whose absolute value of amplitude is 12 mV or higher and whose duration is within 2 sec.

Furthermore, as for a movement of a person on the bed 110, for example, the determining unit 502 determines that the voltage signal indicating the amount of deformation of the connecting frame 130 exceeds the threshold when it is found that a peak is present whose maximum amplitude is -6 mV or lower and whose duration is within 2 sec, as a result of comparison performed by the comparator 402.

The function of the determining unit 502 is implemented when the CPU in the microcomputer configuring the determining mechanism 400 shown in Fig. 4 executes a program, from among the programs stored in a memory such as the ROM and the RAM in the microcomputer, relating to the determination of deformation of the connecting frame 130.

The output unit 505 sends a result of determination made by the determining unit 502, for example, to an indicator lamp not shown. The output unit 505 sends an alarm signal to the alarm 403 as a result of determination, for example, when a pulsed signal is output from the comparator 402. In other words, the alarm signal is output when the amount of deformation of the connecting frame 130 exceeds the threshold.

The function of the output unit 505 is implemented when the CPU in the microcomputer configuring the determining mechanism 400 shown in Fig. 4 executes a program, from among the programs stored in a memory such as the ROM and the RAM in the microcomputer, relating to output of a result of determination.

Next, an example of a waveform pattern of the voltage signal output from the unimorph device 140 is explained with reference to Figs. 6 and 7. Figs. 6 and 7 are graphs illustrating a waveform pattern of the voltage signal that is output from the unimorph device 140. A graph 600 shown in Fig. 6 is an example of a voltage signal originating from a movement of a person getting on the bed 110.

In the graph 600, a vertical axis indicates a voltage value (mV) and a horizontal axis indicates time (sec). A part 601 indicates that a person has got on the bed 110. As described above, positive voltage is generated from the piezoelectric device 301 when the unimorph device 140 is deformed along with the connecting frame 130. The voltage is reversed by the amplifier 303 (impedance conversion device) to appear as a large negative signal at this part 601.

Further, a part 602 indicates a rebound caused when the person gets on the bed 110. Based on such a graph, the threshold in the case of a person getting on the bed 110 is set as, for example, "maximum amplitude: -12 mV" and "duration: 2 sec".

On the other hand, a graph 700 shown in Fig. 7 is an example of a voltage signal originating from a movement of a person getting off the bed 110. A part 701 indicates that a person has moved to an edge of the bed 110. In other words, the part 701 indicates output of a medium negative signal when the person has move toward the connecting frame 130 on which the unimorph device 140 is provided, thereby bending the unimorph device 140. Positive voltage is generated from the piezoelectric device 301 when the unimorph device 140 is bent. The voltage is reversed by the amplifier 303 (impedance conversion device) to appear as the negative voltage at the part 701. Based on this curve, the threshold in the case of a movement of a person on the bed 110 is set as, for example, "maximum amplitude: -6 mV" and "duration: 2 sec".

A part 702 indicates that the person has got off the bed 110. As described above, negative voltage is generated from the piezoelectric device 301 when the unimorph device 140 is warped along with the connecting frame 130 when a person gets off the bed 110. The voltage is reversed by the amplifier 303 (impedance conversion device) to appear as a large positive signal at the part 702. Based on this curve, the threshold in the case of a person getting off the bed 110 is set as, for example, "maximum amplitude: +12 mV" and "duration: 2 sec".

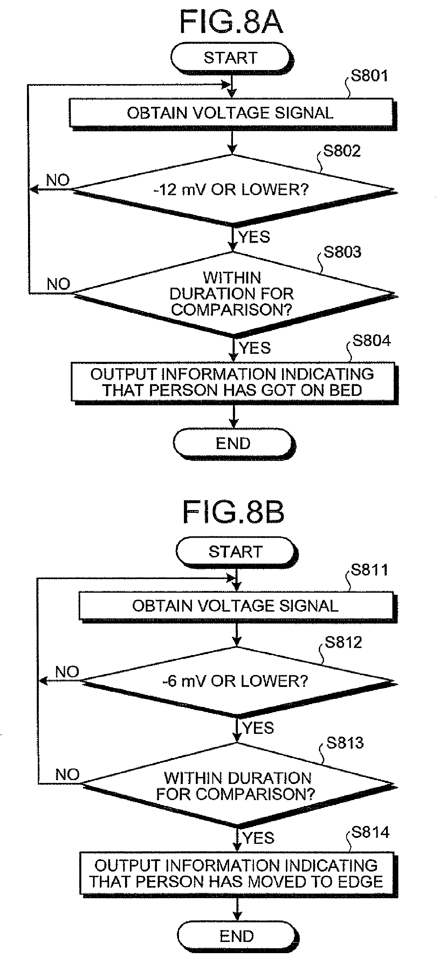

Next, an example of a processing performed by the bed apparatus 100 when a person gets on the bed 110 is explained with reference to Fig. 8A. Fig. 8A is a flowchart of the processing performed by the bed apparatus 100 when a person gets on the bed 110.

As shown in Fig. 8A, first, the detecting unit 501 obtains a voltage signal (step S801). It is then determined whether a voltage value of the obtained voltage signal is equal to or lower than -12 mV (is equal to or exceeds the threshold) (step S802). When it is determined that a voltage value is equal to or lower than -12 mV (is equal to or exceeds the threshold) (step S802: YES), it is determined whether, in the obtained voltage signal, a peak of the voltage signal that fluctuates beyond the threshold has a duration within the duration for comparison (step S803).

When it is determined that the duration of the peak is within the duration for comparison (step S803: YES), information indicating that a person has got on the bed 110 is output (step S804). Thus, a series of the processing is ended.

On the other hand, when it is determined that a voltage value is not equal to or lower than -12 mV (step S802: NO), in other words, when it is determined that a voltage value is not equal to or beyond the threshold, the process returns to step S801. Moreover, when it is determined that the duration of the peak is not within the duration for comparison (step S803: NO), the process returns to step S801.

Fig. 8B is a flowchart of the processing performed by the bed apparatus 100 when a movement of a person on the bed 110 is to be determined. The processing shown in Fig. 8B is performed after it has been determined that a person has got on the bed 110 by the processing shown in Fig. 8A.

As shown in Fig. 8B, first, the detecting unit 501 obtains a voltage signal (step S811). It is then determined whether a voltage value of the obtained voltage signal is equal to or lower than -6 mV (equal to or exceeds the threshold) (step S812). When it is determined that a voltage value is equal to or lower than -6 mV (equal to or exceeds the threshold) (step S812: YES), it is determined whether, in the obtained voltage signal, a peak of the voltage signal that fluctuates beyond the threshold has a duration within the duration for comparison (step S813).

When it is determined that the duration of the peak is within the duration for comparison (step S813: YES), information indicating that a person on the bed 110 has moved to an edge thereof is output (step S814). Thus, a series of the processing is ended.

On the other hand, when it is determined that a voltage value is not equal to or lower than -6 mV (step S812: NO), in other words, when it is determined that a voltage value is not equal to or beyond the threshold, the process returns to step S811. Moreover, when it is determined that the duration of the peak is not within the duration for comparison (step S813: NO), the process returns to step S801.

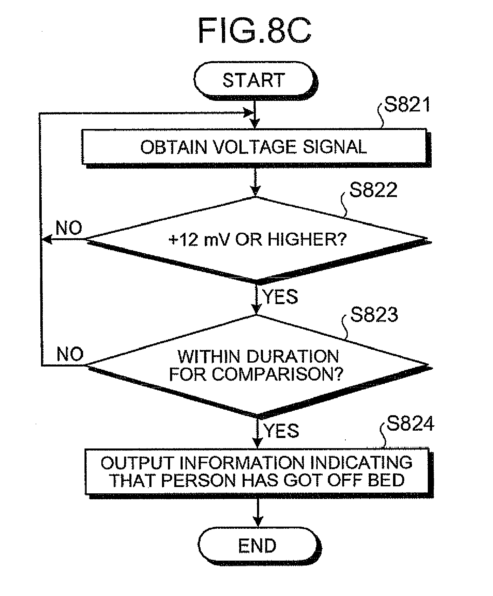

Fig. 8C is a flowchart of the processing performed by the bed apparatus 100 when a person gets off the bed 110. The processing shown in Fig. 8C is performed after it has been determined that a person has got on the bed 110 by the processing shown in Fig. 8A and it has been determined that the person on the bed 110 has moved to the edge thereof by the processing shown in Fig. 8B.

As shown in Fig. 8C, first, the detecting unit 501 obtains a voltage signal (step S821). It is then determined whether a voltage value of the obtained voltage signal is equal to or higher than +12 mV (equal to or exceeds the threshold) (step S822). When it is determined that a voltage value is equal to or higher than +12 mV (equal to or exceeds the threshold) (step S822: YES), it is determined whether, in the obtained voltage signal, a peak of the voltage signal that fluctuates beyond the threshold has a duration within the duration for comparison (step S823).

When it is determined that the duration of the peak is within the duration for comparison (step S823: YES), information indicating that a person has got off the bed 110 is output (step S824). Thus, a series of the processing is ended.

On the other hand, when it is determined that a voltage value is not equal to or higher than +12 mV (step S822: NO), in other words, when it is determined that a voltage value is not equal to or beyond the threshold, the process returns to step S821. Moreover, when it is determined that the duration of the peak is not within the duration for comparison (step S823: NO), the process returns to step S821.

As described above, the bed apparatus 100 according to a first embodiment of the present invention is configured to determine a getting on/off the bed 110 of a person and a movement of a person on the bed 110 based on the unimorph device 140, which is provided on a part of the connecting frame 130 of the bed 110 and continuously detects an amount of deformation of the connecting frame 130, and a result of detection by the unimorph device 140.

Therefore, a getting on/off the bed 110 of a person and a movement of a person on the bed 110 can be determined easily just by detecting an amount of deformation of the connecting frame 130 of the bed 110 without touching the body of a person. Accordingly, it is possible to determine a body movement of a person on the bed 110 easily without causing an unpleasant feeling, restricting movement, or conscious awareness of such monitoring by the person.

Moreover, since the unimorph device 140 is not directly in contact with the body of a person, waterproofing and electrical insulation are not required, thereby enabling a simple structure. In addition, since a person does not lie on the sensor, body pressure distribution is not lost, thereby eliminating concerns of a pressure sore.

Furthermore, as a movement of a person on the bed to an edge thereof can be detected, when a patient that should not get off the bed 110 moves to the edge, it is possible to react before the patient actually gets off the bed 110, or quickly even if the patient has got off the bed 110.

Moreover, if the bed apparatus 100 according to the first embodiment is configured such that the unimorph device 140 is provided at more than one connecting frame 130 to detect deformation of the connecting frames 130, and such that a difference operation value of signals detected by the unimorph devices 140 is detected, an amount of deformation of the connecting frames 130 can be detected in more detail, thereby determining a body movement of a person on the bed 110 in further detail. In addition, a body movement of a person on the bed 110 can be accurately detected even if the bed 110 has a complicated frame structure.

Furthermore, since the unimorph device 140 is used in the bed apparatus 100 according to the first embodiment, a compact and simple structure is achieved, and an amount of deformation of the connecting frame 130 can be accurately detected.

Moreover, according to the bed apparatus 100 of the first embodiment, since a metallic frame for which hysteresis with respect to stress is low and a change over time is small is used in the bed 110, the amount of deformation of the frame of the bed 110 can be accurately detected even when the bed 110 is used for a long time.

While a case where the unimorph device 140 is provided on top of the connecting frame 130 has been explained above, in a case in which the unimorph device 140 is provided at the bottom of the connecting frame 130, the voltage to be extracted and the threshold for comparison are reversed between positive and negative. Specifically, plus and minus signs of values shown in the waveforms and the flowcharts described above are to be reversed. The same is true for a second embodiment and a third embodiment explained below.

Next the bed apparatus 100 and a method of determining a body movement of the bed apparatus 100 according to the second embodiment of the present invention are explained. In the second embodiment, an example of determining a body movement related to awake- and sleeping states of a person on the bed 110 is explained. In the second embodiment, the determining mechanism and the threshold for comparison are different from those of the first embodiment. Like reference characters are used to identify like parts shown in the first embodiment, and explanation therefor is omitted.

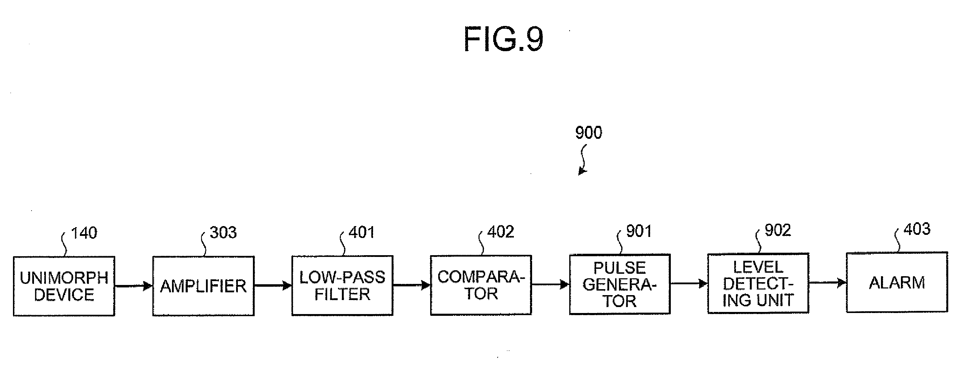

A functional configuration of a determining mechanism 900 is explained with reference to Fig. 9. Fig. 9 is a block diagram of the determining mechanism 900. As shown in Fig. 9, the determining mechanism 900 includes the unimorph device 140, the amplifier 303, the low-pass filter 401, the comparator 402, a pulse generator 901, a level detecting unit 902, and the alarm 403.

The low-pass filter 401 filters for components of 0.1 Hz to 2 Hz from a voltage amplified by the amplifier 303, and the components pass to the comparator 402. Thus, components related to an awake-state or a sleeping state of a person on the bed 110 can be extracted from components of the voltage amplified by the amplifier 303.

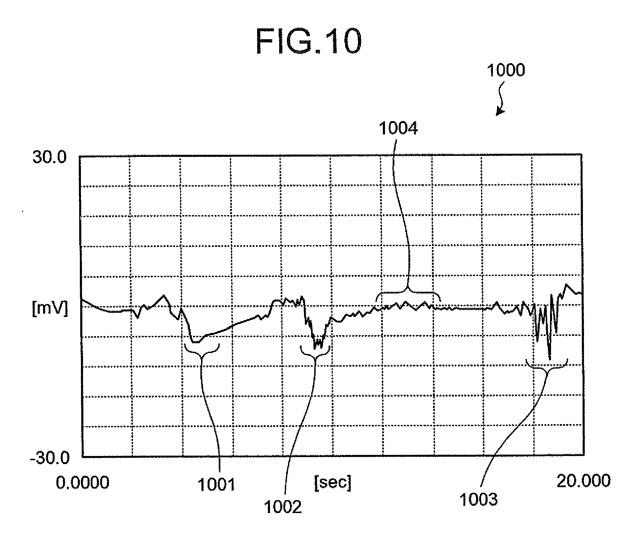

The comparator 402 has a storage area (not shown) to store a threshold used for comparison, and compares the threshold stored in the storage area and a voltage signal that is obtained by filtering with the low-pass filter 401. The comparator 402 sends a signal corresponding to a result of the comparison to the pulse generator 901 downstream. The threshold is set in advance based on waveform patterns (refer to Fig. 10) that are expressed by voltage and time corresponding to awake and sleeping states of a person on the bed 110. The threshold, specifically, is expressed by an amplitude and a duration of a voltage signal.

While the threshold is explained in detail hereinafter with reference to Fig. 10, for example, it is set as "maximum amplitude: -6 mV" and "duration: 2 sec". This threshold corresponds to a voltage signal generated when a person moves limbs or changes position.

The comparator 402 compares, for example, the threshold and the voltage signal that is obtained by filtering with the low-pass filter 401, and sends a result of comparison to the pulse generator 901.

When the signal obtained by filtering exceeds the threshold (specifically, when it is lower than -6 mV), the pulse generator 901 generates, based on the result of comparison sent from the comparator 402, a pulse having a period longer than the lowest frequency detected. The pulse generator 901 superimposes the generated pulse on the signal obtained by filtering for output to the level detecting unit 902 downstream. By superimposing the pulse, when the signal obtained by filtering exceeds the threshold, it is possible to keep the level of the signal output from the pulse generator 901 high.

The pulse generator 901 includes a timing unit (not shown) and based on timing by the timing unit, outputs a low level signal, when a high level signal is not output, for example, for 3 minutes. It is set to 3 minutes because unconscious body movement such as a change of the body position is observed at least once every 3 minutes during an awake-state of healthy people, and if a person does not change body position for 3 minutes, it is assumed that the person is asleep. This timing unit is implemented by the timer 504 in the functional configuration of the bed apparatus 100 shown in Fig. 5.

The level detecting unit 902 detects the level of a signal that is output from the pulse generator 901, and outputs a signal corresponding to a result of the detection. Specifically, when the signal output from the pulse generator 901 is at a high level, the level detecting unit 902 outputs a signal indicating that the person is awake. On the other hand, when the signal output from the pulse generator 901 is at a low level, the level detecting unit 902 outputs a signal indicating that the person is asleep.

The alarm 403 outputs an alarm signal based on the output of the level detecting unit 902. For example, when the level detecting unit 902 outputs the signal indicating that the person is awake, the alarm 403 sends an alarm signal to an indicator lamp (not shown), thereby lighting the indicator lamp to indicate that a person on the bed 110 is awake. Such an indicator lamp is provided, for example, in a management room from which patients are managed in a hospital. Moreover, for example, when the level detecting unit 902 outputs the signal indicating that the person is asleep, the alarm 403 sends an alarm signal to an indicator lamp, thereby lighting the indicator lamp to indicate that a person on the bed 110 is asleep.

The amplifier 303, the low-pass filter 401, the comparator 402, the pulse generator 901, the level detecting unit 902, and the alarm 403 in the determining mechanism 900 can be implemented by a microcomputer that is configured with a ROM that stores programs to realize each function, a CPU that executes various kinds of arithmetic processings based on the programs stored in the ROM, a RAM that functions as a work area of data for the CPU to perform an arithmetic processing, and the like.

Each function of the amplifier 303, the low-pass filter 401, the comparator 402, the pulse generator 901, the level detecting unit 902, and the alarm 403 is not limited to implementation by a single microcomputer, and each function can be implemented, for example, by a combination of microcomputers (circuits) that separately realize each function.

Next, an example of a waveform pattern of the voltage signal output from the unimorph device 140 is explained with reference to Fig. 10. Fig. 10 is a graph illustrating a waveform pattern of the voltage signal output from the unimorph device 140. A graph 1000 shown in Fig. 10 is an example of a voltage signal originating from a body movement of a person on the bed 110.

In the graph 1000, a vertical axis indicates a voltage value (mV) and a horizontal axis indicates time (sec). Parts 1001 to 1003 indicate a gross movement when the person on the bed 110 moves limbs or changes position. As described above, positive voltage is generated from the piezoelectric device 301 when the unimorph device 140 is deformed along with the connecting frame 130. The voltage is reversed by the amplifier 303 (impedance conversion device) to appear as a negative signal.

Based on this curve, a threshold for a gross movement (awake-state) of a person on the bed 110 is set as, for example, "maximum amplitude: -6 mV" and "duration: 2 sec". A part 1004 indicates a slight movement originating from a heartbeat of a person on the bed 110 to be explained in the third embodiment described hereinafter.

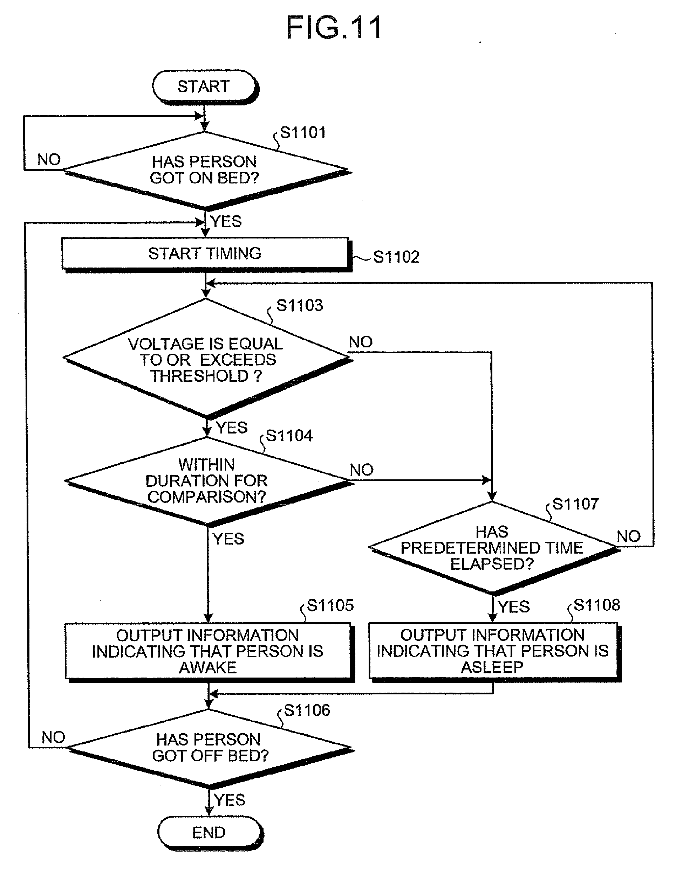

Next, an example of a processing performed by the bed apparatus according to the second embodiment is explained with reference to Fig. 11. Fig. 11 is a flowchart of the processing performed by the bed apparatus according to the second embodiment when it is to be determined whether a person on the bed 110 is awake or asleep.

As shown in Fig. 11, the bed apparatus determines whether a person has got on the bed 110 (step S1101). The determination at step S1101 is made by performing the processing described in the first embodiment (see Fig. 8). At step S1101, waiting occurs until determination that a person has got on the bed 110 is made (step S1101: NO). When it is determined that a person has got on the bed 110 (step S1101: YES), timing begins (step S1102).

A voltage value of a voltage signal that is obtained continuously and the threshold are compared to determine whether a voltage value is equal to or exceeds the threshold (step S1103). Specifically, it is determined whether a voltage value is equal to or lower than -6 mV. When it is determined that a voltage value is equal to or exceeds the threshold (step S1103: YES), it is determined whether, in the obtained voltage signal, a peak of the voltage signal that fluctuates beyond the threshold has a duration within the duration for comparison (step S1104).

When it is determined that the duration of the peak is within the duration for comparison (step S1104: YES), information indicating that the person is awake is output (step S1105). Thereafter, it is determined whether the person has got off the bed 110 (step S1106). The determination at step S1106 is made by performing the processing described in the first embodiment (see Fig. 8). When it is determined that the person has got off the bed 110 (step S1106: YES), a series of the processing is ended.

On the other hand, when it is determined that the person has not got off the bed 110 (step S1106: NO), the process returns to step S1102. Moreover, when it is determined that a voltage value is not equal to or beyond the threshold (step S1103: NO), whether a predetermined time has elapsed is determined (step S1107). The predetermined time is, as described above, 3 minutes during which people unconsciously change body position at least once while awake.

When it is determined that the predetermined time has elapsed (step S1107: YES), information indicating that the person is asleep is output (step S1108), and the process returns to step S1106. On the other hand, when it is determined that the predetermined time has not passed (step S1107: NO), the process returns to step S1103. Moreover, when it is determined that the duration is not within the duration for comparison (step S1104: NO), the process proceeds to step S1107.

As described above, the bed apparatus according to the second embodiment is configured to determine whether a person on the bed 110 is awake or asleep based on the unimorph device 140, which is arranged at a part of the connecting frame 130 of the bed 110 and continuously detects an amount of deformation of the connecting frame 130, and a result of detection by the unimorph device 140.

Therefore, whether a person on the bed 110 is awake or asleep can be determined easily just by detecting an amount of deformation of the connecting frame 130 of the bed 110 without touching the person. Accordingly, it is possible to determine body movement of a person on the bed 110 easily without causing an unpleasant feeling, restricting movement, or conscious awareness of such monitoring by the person.

Moreover, if the bed apparatus 100 according to the second embodiment is configured such that the unimorph device 140 is arranged at more than one connecting frame 130 to detect deformation of the connecting frames 130, and such that a difference operation value of signals detected by the unimorph devices 140 is detected, an amount of deformation of the connecting frames 130 can be detected in more detail, thereby determining a body movement of a person on the bed 110 in further detail. In addition, a body movement of a person on the bed 110 can be accurately detected even if the bed 110 has a complicated frame structure.

Next the bed apparatus 100 and a method of determining a body movement of the bed apparatus 100 according to the third embodiment of the present invention are explained-In the third embodiment, an example of determining a body movement related to a heartbeat of a person on the bed 110 is explained. In the third embodiment, the threshold for comparison is different from that of the second embodiment. Like reference characters are used to identify like parts shown in the first embodiment and the second embodiment, and explanation therefor is omitted. The third embodiment is explained with reference to Figs. 9, 10, and 12.

The determining mechanism according to the third embodiment has the same configuration as the determining mechanism 900 according to the second embodiment shown in Fig. 9. However, each set value is different.

The low-pass filter 401 filters for components of 0.1 Hz to 10 Hz from a voltage amplified by the amplifier 303, and the components pass to the comparator 402. Thus, components originating from a heartbeat of a person on the bed 110 can be extracted from components of the voltage amplified by the amplifier 303.

The comparator 402 has a storage area (not shown) to store a threshold used for comparison, and compares the threshold stored in the storage area and a voltage signal that is obtained by filtering with the low-pass filter 401. The comparator 402 sends a signal corresponding to a result of the comparison to the pulse generator 901 downstream. The threshold is set in advance based on a waveform pattern (refer to 1004 shown in Fig. 10) that is expressed by voltage and time corresponding to a heartbeat of a person on the bed 110. The threshold, specifically, is expressed by an amplitude and a duration of a voltage signal.

Based on a curve of the slight body movement shown in Fig. 10, the threshold for a heartbeat of a person on the bed 110 is set, for example, as "maximum amplitude: 1 mV" and "duration: 0.2 sec". The maximum amplitude is expressed as an absolute value.

The comparator 402 compares, for example, the threshold and the voltage signal that is obtained by the filtering, and sends a result of comparison to the pulse generator 901.

When the signal obtained by filtering is higher than the threshold, the pulse generator 901 generates, based on the result of comparison sent from the comparator 402, a pulse having a period longer than the lowest frequency detected. The pulse generator 901 superimposes the generated pulse on the signal obtained by filtering, for output to the level detecting unit 902 downstream. By superimposing the pulse, when the signal obtained by filtering is larger than the threshold, it is possible to keep the level of the signal output from the pulse generator 901 high.

The pulse generator 901 includes a timing unit (not shown), and based on timing by the timing unit, outputs a low level signal, when a high level signal is not output, for example, for 3 seconds. The heart rate of a human is 30 more than beats per minute. For example, if it is assumed that the heart rate is 20 beats per minute, it can be assumed that a heartbeat is observed at least once every three seconds. In other words, when no heartbeat is observed for 3 seconds, it can be assumed that the heart has stopped beating or the person is not on the bed 110. This timing unit is implemented by the timer 504 in the functional configuration of the bed apparatus 100 shown in Fig. 5.

The level detecting unit 902 detects the level of a signal that is output from the pulse generator 901, and outputs a signal corresponding to a result of the detection. Specifically, when the signal output from the pulse generator 901 is at a high level, the level detecting unit 902 outputs a signal indicating that a person is present (alive) on the bed 110. On the other hand, when the signal output from the pulse generator 901 is at a low level, the level detecting unit 902 outputs a signal indicating cardiac arrest (or that a person is absent from the bed 110).

The alarm 403 outputs an alarm signal based on the output of the level detecting unit 902. For example, when the level detecting unit 902 outputs the signal indicating that the person is present, the alarm 403 sends an alarm signal to an indicator lamp (not shown), thereby lighting the indicator lamp to indicate that a person is present on the bed 110. Such an indicator lamp is provided, for example, in a management room from which patients are managed in a hospital.

Moreover, for example, when the level detecting unit 902 outputs the signal indicating that the person is absent or indicating cardiac arrest, the alarm 403 sends an alarm signal to an indicator lamp, thereby lighting the indicator lamp to indicate cardiac arrest (or that a person is absent from the bed 110).

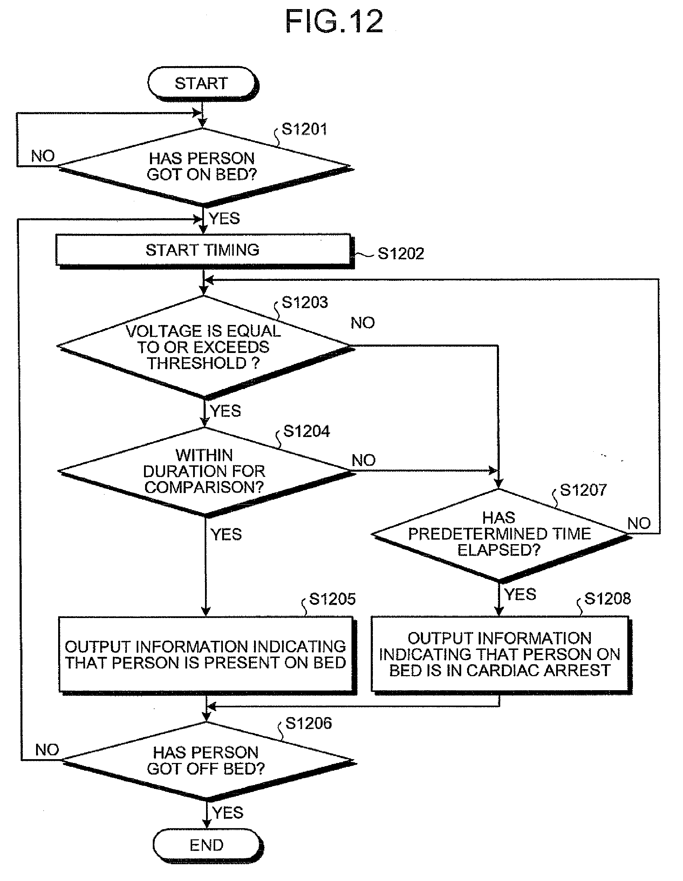

Next, an example of a processing performed by the bed apparatus according to the third embodiment when a state of heartbeat of a person on the bed 110 is determined is explained with reference to Fig. 12. Fig. 12 is a flowchart of the processing performed by the bed apparatus according to the third embodiment when a heartbeat state of a person on the bed 110 is to be determined.

As shown in Fig. 12, the bed apparatus determines whether a person has got on the bed 110 (step S1201). The determination at step S1201 is made by performing the processing described in the first embodiment (see Fig. 8). At step S1201, waiting occurs until determination that a person has got on the bed 110 is made (step S1201: NO). When it is determined that a person has got on the bed 110 (step S1201: YES), timing begins (step S1202).

A voltage value of a voltage signal that is obtained continuously and the threshold are compared, to determine whether a voltage value is equal to or exceeds the threshold (step S1203). When it is determined that a voltage value is equal to or exceeds the threshold (step S1203: YES), it is determined whether, in the obtained voltage signal, a peak of the voltage signal that fluctuates beyond the threshold has duration within the duration for comparison (step S1204).

When it is determined that the duration of the peak is within the duration for comparison (step S1204: YES), information indicating that the person is present on the bed 110 is output (step S1205). Thereafter, it is determined whether the person has got off the bed 110 (step S1206). The determination at step S1106 is made by performing the processing described in the first embodiment (see Fig. 8). When it is determined that the person has got off the bed 110 (step S1206: YES), a series of the processing is ended.

On the other hand, when it is determined that the person has not got off the bed 110 (step S1206: NO), the process returns to step S1202. Moreover, when it is determined that a voltage value is not equal to or beyond the threshold (step S1203: NO), whether a predetermined time has elapsed is determined (step S1207). The predetermined time is, as described above, 3 seconds, for example.

When it is determined that the predetermined time has elapsed (step S1107: YES), information indicating that the person on the bed 110 is in cardiac arrest is output (step S1208), and a series of the processing is ended. On the other hand, when it is determined that the predetermined time has not elapsed (step S1207: NO), the process returns to step S1203. Moreover, when it is determined that the duration is not within the duration for comparison (step S1204: NO), the process proceeds to step S1207.

For example, even if it is determined that the person has got off the bed 110 at step S1206 because of a strong movement of the person on the bed 110, when it is determined that the person has a heartbeat (is present) based on the voltage signal that is obtained continuously, the processing described above can be restarted.

As described above, the bed apparatus according to the third embodiment is configured to determine a body movement related to a heartbeat of a person on the bed 110 based on the unimorph device 140 that is provided on a part of the connecting frame 130 of the bed 110 and continuously detects an amount of deformation of the connecting frame 130, and a result of detection by the unimorph device 140.

Therefore, a state of the heartbeat of a person on the bed 110 can be determined easily just by detecting an amount of deformation of the connecting frame 130 of the bed 110 without touching the person. Accordingly, it is possible to determine a body movement of a person on the bed 110 easily without causing an unpleasant feeling, restricting movement, or conscious awareness of such monitoring by the person.

Moreover, if the bed apparatus is configured such that the unimorph device 140 is provided at more than one connecting frame 130 to detect deformation of the connecting frames 130, and such that a difference operation value of signals detected by the unimorph devices 140 is detected, an amount of deformation of the connecting frames 130 can be detected in more detail, thereby determining a body movement of a person on the bed in further detail. In addition, a body movement of a person on the bed 110 can be accurately detected even if the bed 110 has a complicated frame structure.

Although the first to the third embodiments described above are explained separately for convenience, it is, of course, possible to embody all at the same time.

As described above, according to the bed apparatus and the method of determining a body movement of the bed apparatus according to the present invention, a body movement of a person on a bed can be determined easily, without causing an unpleasant feeling, restricting movement, or conscious awareness of such monitoring by the person.

Although the invention has been described with respect to a specific embodiment for a complete and clear disclosure, the appended claims are not to be thus limited but are to be construed as embodying all modifications and alternative constructions that may occur to one skilled in the art which fairly fall within the basic teaching herein set forth.

The present document incorporates by reference the entire contents of Japanese priority document, 2007-294008 filed in Japan on November 13, 2007.

## Claims

1. A bed apparatus comprising:
a frame of a bed;
a deformation-amount detecting unit that is disposed on a part of the frame and continuously detects an amount of deformation of the frame;
a determining unit that determines a body movement of a person on the bed based on a result of detection by the deformation-amount detecting unit; and
an output unit that outputs a result of determination made by the determining unit.

2. The bed apparatus according to claim 1, wherein the determining unit determines whether the person is getting on or off the bed.

3. The bed apparatus according to claim 1, wherein the determining unit determines whether the person is changing body position.

4. The bed apparatus according to claim 1, wherein the determining unit determines whether the person is awake or asleep.

5. The bed apparatus according to claim 1, wherein the determining unit determines a heartbeat state of the person.

6. The bed apparatus according to claim 1, wherein
the frame includes a plurality of legs and connecting members that connect the legs, and
the deformation-amount detecting unit is disposed on the connecting members and detects deformation of the connecting members.

7. The bed apparatus according to claim 1, wherein the deformation-amount detecting unit includes
a disc-shaped piezoelectric device that is deformed as a result of an external force applied to the frame, and
an impedance conversion device that performs impedance conversion on a voltage output from the piezoelectric device.

8. The bed apparatus according to claim 1, wherein the frame is a metallic frame.

9. A body movement determining method for a bed apparatus that includes a frame of a bed and a deformation-amount detecting unit disposed on a part of the frame, the body movement determining method comprising:
detecting continuously an amount of deformation of the frame;
determining a body movement of a person on the bed based on a result of detection at the detecting; and
outputting a result of determination made by the determining unit.
